# EUROPEAN PATENT APPLICATION

(11) **EP 2 614 846 A1**
(43) Date of publication of application: **17.07.2013**
(21) Application number: 11819398.6
(22) Date of filing: 16.08.2011
(51) Int. Cl.: A61L 31/06, A61L 31/04, A61L 31/18, A61F 2/02

(54) **EMBOLIC AGENT AND PREPARATION METHOD THEREFOR**

(30) Priority: 27.08.2010 CN 201010267116
(71) Applicant: Shanghai MicroPort Medical (Group) Co., Ltd., Shanghai 201203 (CN)
(72) Inventor: LI, Yu, Shanghai 201203 (CN); KANG, Yahong, Shanghai 201203 (CN); JIN, Qiaorong, Shanghai 201203 (CN); XIE, Zhiyong, Shanghai 201203 (CN); LUO, Qiyi, Shanghai 201203 (CN)
(74) Representative: FDST Patentanwälte
(86) International application number: PCT/CN2011/078457
(87) International publication number: WO 2012/025023

(57) **Abstract**

An embolic agent and a preparation method therefore are disclosed. The embolic agent comprises a liquid embolic material, a degradable polymer, and a contrast agent. The preparation method comprises physically blending a degradable polymer and a liquid embolic material, or chemically bonding the degradable polymer to the liquid embolic material, to obtain a first solution; adding a contrast agent to the first solution, to obtain the embolic agent. Due to the gradual degradation of the degradable polymer, an embolic body forms a system with a porous structure, thereby lowering the density of the embolic agent and effectively relieving the mass effect.

## Description

### Cross Reference to Related Application

This application claims the priority to the application No. 201010267116.3, filed on August 27, 2010 to the Chinese State Intellectual Property Office, titled "Embolic agent and preparation method therefor", and incorporated herein by reference in its entirety.

### Technical Field

The present invention is in the field of material technology, and is particularly directed to an embolic agent and a preparation method therefor.

### Background

Aneurysms may be formed in arteriovenous vessels as a result of the influence of internal and external factors including mechanical damage, arteriosclerosis, hypertension, vascular smooth muscle hyperplasia, bacteria or virus infection, venous valve disease induction or blood flow impact. There are arterial aneurysms and venous aneurysms. An aneurysm with round bulging walls formed intracranially is called cerebral arterial aneurysm, the rupture of which easily induces a subarachnoid hemorrhage leading to a stroke in the patient. What's even worse is that 80% or more patients will die after rupture of a recurrent cerebral arterial aneurysm. Therefore, a best treatment solution is to embolize or occlude the cerebral arterial aneurysm at the earliest possible stage of its occurrence.

Among methods of embolizing cerebral arterial aneurysms, one generally used is through surgical intervention including snipping and ligating the parent artery of the aneurysm, and closing the base of the aneurysm with a clip so as to block the impact of the blood flow to the aneurysm. However, this method is time consuming and may cause damage to the aneurysm. In recent years, with the development of vascular imaging, traditional surgical treatment has been gradually replaced by a method of using platinum coils planted into the cerebral aneurysm cavity to occlude and embolize the aneurysm, although this method has low rate of embolization and tends to form a large-scale thrombus causing brain infarcts.

Up to now, a variety of embolic materials mainly classified as solid and liquid embolic materials have been published in the art. Liquid embolic materials, such as a water insoluble polymer and cyanoacrylate etc., can be injected directly into the cavity of the aneurysm to achieve permanent occlusion whereas leaving no space between the wall of the aneurysm and the embolic material and thereby are suitable for aneurysms with various shapes and dimensions of the cavity. Meanwhile, as being easy to handle and can be injected into the blood vessel through a microcatheter, liquid embolic materials are widely used for endovascular therapies. However, due to a greater density of the embolic body formed when using liquid embolic materials disclosed in the prior art to solidify in the blood, a huge mass effect can be caused when a complete embolization of the aneurysm is achieved and this brings extremely uncomfortable feelings to the patient.

### Content of the Invention

Accordingly, the purpose of the present invention is to provide an embolic agent with alleviated mass effect and a preparation method therefor.

The present invention provides an embolic agent comprising a liquid embolic material, a degradable polymer, and a contrast agent.

Preferably, the degradable polymer comprises one or more polymers selected from polylactic acid, polyglycolide, polycaprolactone, polyanhydrides, poly(1,2-propylene fumarate), polyphosphazenes, polymer derived from L-tyrosine, polyorthoesters , amino acid based polymer, chitin and derivative thereof, chitosan and derivative thereof, hyaluronic acid and derivative thereof, chondroitin sulfate and derivative thereof, collagen and derivative thereof, gelatin and derivative thereof, agar and derivative thereof, fibrin and derivative thereof, and silk protein and derivative thereof.

Preferably, the degradable polymer is polylactic acid.

Preferably, the molar ratio of the liquid embolic material to the degradable polymer is 1~20:10.

Preferably, the molar ratio of the liquid embolic material to the degradable polymer is 1~10:10.

Preferably, the degradable polymer has a number averaged molecular weight of 10,000~100,000.

Preferably, the degradable polymer has a number averaged molecular weight of 50,000~80,000.

Preferably, the contrast agent comprises one or more of the following substances: iodide, bismuth compound, tantalum powder and gold powder.

The present invention further provides a preparation method for an embolic agent, comprising:
physically blending a degradable polymer and a liquid embolic material, or chemically bonding the degradable polymer to the liquid embolic material, to obtain a first solution; and
adding a contrast agent to the first solution to obtain the embolic agent.

Preferably, the degradable polymer comprises one or more polymers selected from polylactic acid, polyglycolide, polycaprolactone, polyanhydrides, poly(1,2-propylene fumarate), polyphosphazenes, polymer derived from L-tyrosine, polyorthoesters, amino acid based polymer, chitin and derivative thereof, chitosan and derivative thereof, hyaluronic acid and derivative thereof, chondroitin sulfate and derivative thereof, collagen and derivative thereof, gelatin and derivative thereof, agar and derivative thereof, fibrin and derivative thereof, and silk protein and derivative thereof.

As can be seen from the above technical solutions, the present invention provides an embolic agent and a preparation method therefor, wherein the embolic agent comprises a liquid embolic material, a degradable polymer, and a contrast agent. After being injected into the cavity of the aneurysm, the embolic agent can solidify in the blood and fill the predetermined region stably so as to fulfill the purpose of aneurysm embolization. In addition, gradual degradation of the degradable polymer renders the embolic body a porous structure system reducing the density of the embolic agent and thereby alleviating the mass effect effectively. Furthermore, the finally resultant porous structure permits attachment and growth of tissue cells upon it, promoting cell differentiation and proliferation, and accelerating vascular endothelialization. As a result, possible blood recanalization after embolization is effectively prevented.

### Detailed Description of the Invention

Technical solutions in the examples of the present invention are described hereafter clearly and completely. It is obvious that the examples described represent only part but not all of the examples of the present invention. Any other examples obtained by those skilled in the art on the basis of the examples in the present invention without any inventive efforts fall into the scope of protection of the present invention.

The present invention discloses an embolic agent comprising a liquid embolic material, a degradable polymer, and a contrast agent.

The liquid embolic material can be an adhesive embolic material or a non-adhesive embolic material. The adhesive embolic material preferably includes a cyanoacrylate compound which can polymerize and form a permanent mass in the blood serving as an embolism. The non-adhesive embolic material is preferably formulated by dissolving a water insoluble macromolecular polymer into certain kind of water soluble organic solvent so that the polymer will precipitate out to embolize the aneurysm when the material is in contact with a water soluble solution.

The degradable polymer can be a synthetic or a natural degradable polymer which preferably comprises one or more polymers selected from polylactic acid, polyglycolide, polycaprolactone, polyanhydrides, poly(1,2-propylene fumarate), polyphosphazenes, polymer derived from L-tyrosine, polyorthodesters, amino acid based polymer, chitin and derivative thereof, chitosan and derivative thereof, hyaluronic acid and derivative thereof, chondroitin sulfate and derivative thereof, collagen and derivative thereof, gelatin and derivative thereof, agar and derivative thereof, fibrin and derivative thereof, and silk protein and derivative thereof. The number averaged molecular weight of the degradable polymer is preferably 30,000~100,000, more preferably 50,000~80,000 and most preferably 55,000~65,000.

According to the present invention, the embolic agent can be injected directly into the aneurysm cavity wherein it solidifies in the blood and fills the predetermined region stably so as to fulfill the purpose of aneurysm embolization. The degradable polymer degrades into carbon dioxide and water, providing the embolic body with a porous structure which lowers the density of the embolic body to a great extent. Therefore, through the gradual degradation of the degradable polymer, the embolic agent of the present invention can alleviate the mass effect by lowering down the density of the embolic body. In addition, the finally resultant porous structure permits attachment and growth of tissue cells upon it, promoting cell differentiation and proliferation and accelerating vascular endothelialization. As a result, possible blood recanalization after embolization is effectively prevented.

The molar ratio of the liquid embolic material to the degradable polymer is preferably 1~20:10, and more preferably 1~10:10.

The embolic agent provided in the present invention is admixed with a contrast agent used in the angiography to advantageously benefit the observation with naked eyes of the embolization of the aneurysm in a fluorescent X-ray photography technique, during or after a surgical operation. The contrast agent comprises one or more of the following substances: iodide, bismuth compound, tantalum powder and gold powder. The iodide includes preferably one or more of the following substances: 6-triiodobenzoic acid, sodium 6-triiodobenzoate, iodotitanate, metrizoic acid, iodamide, ioxaglic acid, iopamidol acid, iohexol and iotrolan. The bismuth compound can be bismuth trioxide. The amount of the contrast agent to be added in the present invention is not specifically required, preferably an amount commonly known by a skilled in the art.

The present invention further provides a preparation method for an embolic agent comprising:
physically blending a degradable polymer and a liquid embolic material, or chemically bonding the degradable polymer to the liquid embolic material, to obtain a first solution; and
adding a contrast agent to the first solution to obtain the embolic agent.

According to the present invention, the process of physically blending the degradable polymer and the liquid embolic material or chemically bonding the degradable polymer to the liquid embolic material is conducted preferably at a temperature of 40~80°C and more preferably 50~70°C. Said process is further conducted preferably under the protection of argon gas, preferably with stirring for 1~12h and even more preferably 2~10h, so as to achieve a thorough mix. The process of adding the contrast agent to the first solution is conducted preferably under the protection of argon gas, preferably with stirring but with no specific requisition on the stirring time as long as the mixture obtained is homogenous.

According to the present invention, the obtained embolic agent is preferably kept in a container sealed and sterilized for storage.

To further illustrate the technical solutions of the present invention, preferred embodiments of the invention is described below with reference to the following examples. It should be understood that these descriptions are only used to further illustrate the features and advantages of the invention and should not be construed as a limit to the claims of the invention.

### Specific Mode For Carrying Out The Invention

### Example 1

A solution formed by adding 10g ethylene-vinyl alcohol polymer (EVOL) with a number averaged molecular weight of 60,000 into 100ml dimethyl sulfoxide (DMSO) is stirred at 50°C in an argon gas atmosphere for 2h. The solution is further added with 3g polylactic acid with a number averaged molecular weight of 150,000 and stirred at 70°C in an argon gas atmosphere for 5h to obtain a homogenous solution. The resulted homogenous solution is added with 30g tantalum powder and stirred till a uniform dispersion is achieved. The final product is then transferred into a container, sealed and sterilized for storage.

### Example 2

A solution formed by adding 10g cellulose acetate polymer (CAP) with a number averaged molecular weight of 60,000 into 100ml DMSO is stirred at 50°C in an argon gas atmosphere for 2h. The solution is further added with 3g poly(lactide-co-glycolide) with a number averaged molecular weight of 150,000 and stirred at 70°C in an argon gas atmosphere for 5h to obtain a homogenous solution. The resulted homogenous solution is added with 30g tantalum powder and stirred till a uniform dispersion is achieved. The final product is then transferred into a container, sealed and sterilized for storage.

### Example 3

A solution formed by adding 10g methacrylic acid copolymer (Eudrsgit-E) with a number averaged molecular weight of 60,000 to 100ml dry ethanol and iopromide is stirred at 50°C in an argon gas atmosphere for 2h. The solution is further added with 3g polyglycolic acid with a number averaged molecular weight of 150,000 and stirred at 70°C in an argon gas atmosphere for 5h to obtain a homogenous solution. The resulted homogenous solution is added with 30g tantalum powder and stirred till a uniform dispersion is achieved. The final product is then transferred into a container, sealed and sterilized for storage.

### Example 4

A solution formed by dispersing 3g polycaprolactone with a number averaged molecular weight of 10,000 into the monomer of n-butyl cyanoacrylate (NBCA) is stirred at 50°C in an argon gas atmosphere for 5h to give a homogenous solution. The resulted homogenous solution is added with 30g tantalum powder and stirred till a uniform dispersion is achieved. The final product is then transferred into a container, sealed and sterilized for storage.

### Example 5

A solution formed by adding 2mol chondroitin sulfate into 10g monomer of n-pentyl cyanoacrylate is stirred at room temperature in an argon gas atmosphere for 12h and then distilled under vacuum to produce a uniform solution which is slightly yellow. The slightly yellow solution is added with 25g nano-tantalum powder and stirred till a uniform dispersion is achieved. The final product is then transferred into a container, sealed and sterilized for storage.

### Example 6

A solution formed by adding 2mol polyglycolide into 10g monomer of n-hexyl cyanoacrylate is stirred at room temperature in an argon gas atmosphere for 12h and then distilled under vacuum to produce a uniform solution which is slightly yellow. The slightly yellow solution is added with 25g nano-tantalum powder and stirred till a uniform dispersion is achieved. The final product is then transferred into a container, sealed and sterilized for storage.

### Example 7

A solution formed by adding 2mol polylactic acid into 10g monomer of n-octyl cyanoacrylate is stirred at room temperature in an argon gas atmosphere for 12h and then distilled under vacuum to produce a uniform solution which is slightly yellow. The slightly yellow solution is added with 25g nano-tantalum powder and stirred till a uniform dispersion is achieved. The final product is then transferred into a container, sealed and sterilized for storage.

When an embolic agent prepared in examples 1-7 is injected into freshly withdrawn blood, a sponge-like precipitate which gets firmer and denser gradually from inside to outside, is formed immediately. In addition, as the degradable polymer degrades, a precipitate with porous structure will finally be formed. It is found that when taken out of the blood, the sponge-like polymer provides a soft feel. Therefore, it is indicated by the experimental result that the embolic agent prepared according to the preparation method provided by the invention can solidify quickly and form a soft structure which is sponge-like and porous.

As can be seen from the above examples, the present invention provides an embolic agent and a preparation method therefor, wherein the embolic agent comprises a liquid embolic material, a degradable polymer, and a contrast agent. After being injected into the cavity of the aneurysm, the embolic agent can solidify in the blood and fill the predetermined region stably so as to fulfill the purpose of aneurysm embolization. In addition, gradual degradation of the degradable polymer renders the embolic body a porous structure system reducing the density of the embolic agent and thereby alleviating the mass effect effectively. Furthermore, the finally resultant porous structure permits attachment and growth of tissue cells upon it, promoting cell differentiation and proliferation and accelerating vascular endothelialization. As a result, possible blood recanalization after embolization is effectively prevented.

Those skilled in the art will be able to implement and use the present invention based on the above description about the disclosed examples. It is obvious to those skilled in the art that there are various modifications available to these examples and the general principles defined in the invention can be utilized in other examples without departing from the sprit or scope of the present invention. Therefore, the present invention will not be limited by the examples demonstrated herein but shall cover the greatest scope in line with the principles and novel features disclosed herein.

## Claims

1. An embolic agent comprising a liquid embolic material, a degradable polymer, and a contrast agent.

2. The embolic agent according to claim 1, wherein the degradable polymer comprises one or more polymers selected from polylactic acid, polyglycolide, polycaprolactone, polyanhydrides, poly(1,2-propylene fumarate), polyphosphazenes, polymer derived from L-tyrosine, polyorthodesters, amino acid based polymer, chitin and derivative thereof, chitosan and derivative thereof, hyaluronic acid and derivative thereof, chondroitin sulfate and derivative thereof, collagen and derivative thereof, gelatin and derivative thereof, agar and derivative thereof, fibrin and derivative thereof, and silk protein and derivative thereof.

3. The embolic agent according to claim 2, wherein the degradable polymer is polylactic acid.

4. The embolic agent according to claim 1, wherein the molar ratio of the liquid embolic material to the degradable polymer is 1~20:10.

5. The embolic agent according to claim 4, wherein the molar ratio of the liquid embolic material to the degradable polymer is 1~10:10.

6. The embolic agent according to any one of claims 1-5, wherein the degradable polymer has a number averaged molecular weight of 10,000~100,000.

7. The embolic agent according to claim 6, wherein the degradable polymer has a number averaged molecular weight of 50,000~80,000.

8. The embolic agent according to any one of claims 1-5, wherein the contrast agent comprises one or more of the following substances: iodide, bismuth compound, tantalum powder and gold powder.

9. A preparation method for an embolic agent, comprising:
physically blending a degradable polymer and a liquid embolic material, or chemically bonding the degradable polymer to the liquid embolic material, to obtain a first solution; and
adding a contrast agent to the first solution to obtain the embolic agent.

10. The preparation method according to claim 9, wherein the degradable polymer comprises one or more polymers selected from polylactic acid, polyglycolide, polycaprolactone, polyanhydride, poly(1,2-propylene fumarate), polyphosphazene, a polymer derived from L-tyrosine, polyorthodester, amino acid based polymer, chitin and derivative thereof, chitosan and derivative thereof, hyaluronic acid and derivative thereof, chondroitin sulfate and derivative thereof, collagen and derivative thereof, gelatin and derivative thereof, agar and derivative thereof, fibrin and derivative thereof, and silk protein and derivative thereof.
